(19) Europäisches Patentamt
European Patent Office
Office européen des brevets

(11) **EP 2 680 957 B1**

(12) **EUROPÄISCHE PATENTSCHRIFT**

(45) Veröffentlichungstag und Bekanntmachung des Hinweises auf die Patenterteilung:
**08.04.2015 Patentblatt 2015/15**

(21) Anmeldenummer: **12706051.5**

(22) Anmeldetag: **29.02.2012**

(51) Int Cl.:
***B01F 5/04*** *(2006.01)*     ***C07C 51/31*** *(2006.01)*
***C07C 51/14*** *(2006.01)*

(86) Internationale Anmeldenummer:
**PCT/EP2012/053391**

(87) Internationale Veröffentlichungsnummer:
**WO 2012/117003 (07.09.2012 Gazette 2012/36)**

(54) **VERFAHREN UND VORRICHTUNG ZUR VERMISCHUNG ZWEIER FLUIDSTRÖME**

METHOD AND DEVICE FOR MIXING TWO FLUID FLOWS

PROCÉDÉ ET DISPOSITIF DE MÉLANGE DE DEUX COURANTS DE FLUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.03.2011 EP 11156408**

(43) Veröffentlichungstag der Anmeldung:
**08.01.2014 Patentblatt 2014/02**

(73) Patentinhaber: **BASF SE**
**67056 Ludwigshafen (DE)**

(72) Erfinder:
• **HEINEN, Kerstin**
**64653 Lorsch (DE)**
• **KARBACH, Stefan**
**67434 Neustadt (DE)**
• **DAMES, Burkhardt**
**64646 Heppenheim (DE)**
• **HAIP, Wolfgang**
**67258 Heßheim (DE)**
• **GERLINGER, Wolfgang**
**67117 Limburgerhof (DE)**
• **MATTKE, Torsten**
**67251 Freinsheim (DE)**

(56) Entgegenhaltungen:
EP-A- 1 467 018    DE-A1- 3 728 557
FR-A- 1 211 889

**Beschreibung**

Die vorliegende Erfindung betrifft eine verbesserte Vorrichtung zur Vermischung zweier Fluidströme 1 und 2 sowie ein Verfahren zur Vermischung von zwei Fluidströmen unter Verwendung der erfindungsgemäßen Vorrichtung. Weiterhin betrifft sie ein verbessertes Verfahren zur Herstellung von Adipinsäure unter Verwendung der erfindungsgemäßen Vorrichtung.

Für die Vermischung zweier Fluidströme sind zahlreiche technische Lösungen bekannt. Unter Fluidströmen im Sinne dieser Erfindung versteht man hierbei allgemein Stoffströme, welche unter den vorherrschenden Bedingungen flüssig oder gasförmig vorliegen, wobei die vorherrschenden Bedingungen hinsichtlich allgemein relevanter Parameter wie Druck oder Temperatur aufgrund unterschiedlichster technischer Anwendungen stark variieren können. Hierbei kann ein Fluidstrom auch eine Mischung aus mehreren unterschiedlichen Stoffen darstellen, die sich alle im gleichen Aggregatzustand (flüssig oder gasförmig) befinden. So werden beispielsweise flüssige Edukte in kontinuierlich durchströmten Reaktorgeometrien mittels eines im Querstrom angebrachten Rohr- und Ringverteilers beigemischt. Weitere bekannte Lösungen sind T-Stücke, statische Mischer oder Kombinationen mehrerer Verteiler (Paul, E.L., Handbook of Industrial Mixing, Jon Wiley & Sons, 2004, p. 391 - 452). In DE 10 2008 037 374 A1 wird ein Ringverteiler beschrieben, bei dem ein torusförmiger Ring mit Löchern in einem Gasbrenner für die Mischung mehrerer Gasströme verwendet wird.

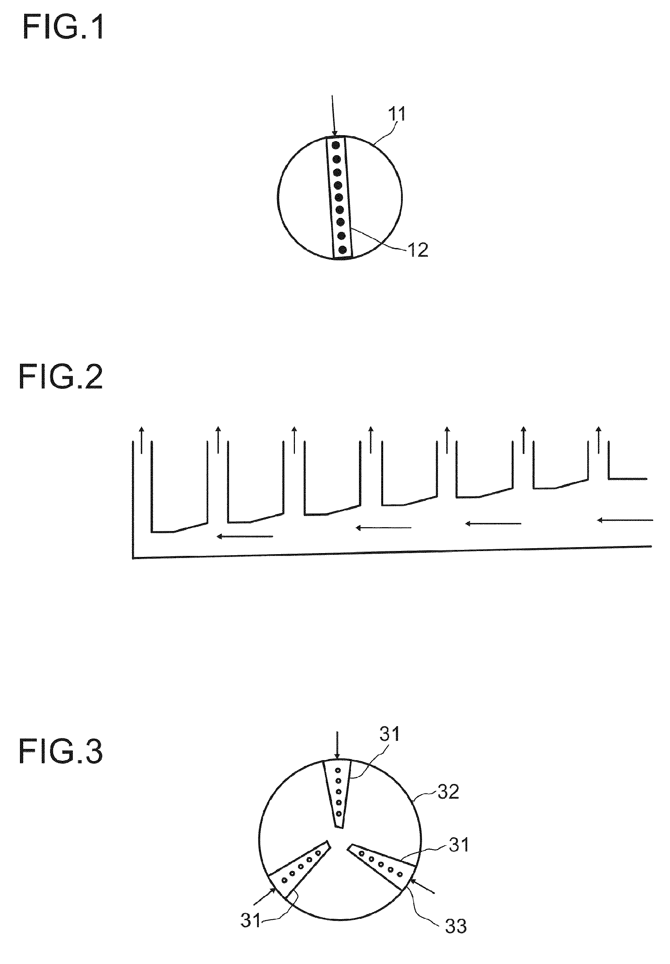

Wird ein Edukt (Fluid) in größerer Menge benötigt als die anderen Edukte (Fluide), wird das Edukt mit dem größten Volumenstrom (im Folgenden mit Fluidstrom 1 bezeichnet) üblicherweise in einer Rohrleitungsgeometrie dem Reaktionsteil zugeführt, der kleinere Eduktvolumenstrom wird dann z.B. durch einen eingesteckten Rohrverteiler im Querstrom zudosiert wie in Figur 1 schematisch dargestellt (im Folgenden mit Fluidstrom 2 bezeichnet). Hier sieht man den Schnitt durch ein Rohr (11), in welches eine Lanze (12) mit Bohrungen zur Verteilung des zweiten Fluidstromes angebracht ist. Bekannte Rohr- oder Ringverteiler bestehen meist aus kreisförmigen Querschnitten mit einer oder mehrerer Lochreihen zur Verteilung des einzumischenden Volumenstroms in den Hauptstrom. Um eine gleichmäßige Aufteilung des Flüssigkeitsvolumenstroms auf alle Löcher zu gewährleisten, muss das Querschnittsverhältnis des Rohrverteilers zur Summe der Lochflächen ausreichend groß sein (Perry's Chemical Engineers Handbook, 8th Edition, Mc Graw Hill, 2008, p. 6-32 - 6-33). Andernfalls erhöht sich durch den in Strömungsrichtung des Verteilers von Loch zu Loch abnehmenden Massenstrom der statische Druck im Verteiler und somit die zur Verfügung stehende Triebkraft zur Ausströmung aus den Löchern. Dies bewirkt eine bevorzugte Durchströmung der in Strömungsrichtung betrachtet stromabwärts liegenden Löcher gegenüber Löchern, die weiter stromaufwärts im Verteiler liegen.

Bei Rohrverteilern ist eine bekannte Möglichkeit die unstetige Anpassung des Strömungsquerschnittes im Verteilerrohr, so dass entlang einer Stromlinie der statische Druck möglichst konstant bleibt. Dies ist in Figur 2 dargestellt. Die Pfeile symbolisieren die Strömungsrichtung des Fluids und man kann die Verringerung des Strömungsquerschnittes in Strömungsrichtung erkennen.

Weiterhin ist die Anpassung der Lochdurchmesser bei konstanter Verteilerquerschnittsfläche bekannt, so dass der Lochdruckverlust in Strömungsrichtung entsprechend dem Anstieg des statischen Drucks im Verteilerrohr ebenfalls zunimmt. Dies kann auch in Form eines Schlitzes realisiert werden, dessen Breite über die Länge veränderlich ist. Eine weitere übliche Alternative ist die Variation des Länge zu Durchmesserverhältnisses der sich an den Verteiler anschließenden Seitenrohre, so dass der Druckverlust in den Seitenrohren an den Anstieg des statischen Drucks im Hauptrohr angepasst wird (Chen, A.W. Sparrow, E.M., Journal Fluid. Eng., Vol. 131 2009, p. 1-9).

Wenn die zu mischenden Fluide an einer exotherme Reaktion beteiligt sind, ist ein möglichst geringes Volumen der Verteilergeometrie meist vorteilhaft. Da eine Rückströmung in die Verteilergeometrie und unkontrollierte Reaktion der Edukte meist nicht verhindert werden kann, ist die Begrenzung des Reaktionsvolumens im Verteiler und bis zur nächsten stromaufwärts liegenden Sicherheitseinrichtung (z.B. eine Rückschlagklappe) vorteilhaft.

In der DE 10 2007 054 770 A1 wird eine Anordnung in einem flüssigkeitsführenden Hohlquerschnitt, insbesondere eine wasserführende Leitung, beschrieben, in dem ein Verdrängerelement eingebaut ist, welches den für die Flüssigkeit zu Verfügung stehenden Querschnitt begrenzt. Damit soll die Stabilität der Leitung bei temperaturbedingter Volumenänderung des Fluids (Erstarren/ Gefrieren) erhöht werden.

In der EP 1 354 866 B1 wird ein Verfahren zur Herstellung von Alkandicarbonsäure beschrieben, bei welchem eine Mischapparatur eingesetzt wird.

In der FR 1 211 889-A wird eine Vorrichtung gemäß dem Oberbegriff des Anspruchs 1 zur Vermischung eines Fluidstromes mit einem anderen Fluidstrom offenbart.

Die bekannten Vorrichtungen zur Vermischung weisen jedoch Nachteile auf. So ergeben sich bei der Vermischung der beiden Fluide immer wieder unerwünschte Inhomogenitäten und zur Erreichung einer zufriedenstellenden Verteilungswirkung ist ein relativ hoher volumenspezifischer Druckverlust aufzubringen, welcher die Effektivität des Mischverfahrens beeinträchtigt. Unter Verteilungswirkung im Sinne dieser Erfindung versteht man die auf den Mittelwert des ideal durchmischten Zustands bezogene Breite der Konzentrationsverteilung des über den Verteiler in einen anderen Raum austretenden Fluids. Zur Beschreibung der Breite der Konzentrationsverteilung wird im Allgemeinen die Standardabweichung verwendet. Eine gute Verteilungswirkung bezeichnet einen Zustand mit einer geringen Breite der Konzentrationsverteilung an einem bestimmten Ort im Verhältnis zum Mittelwert des ideal durchmischten Zustands, d.h. eine kleine Standardabweichung. Als gut durchmischt wird im Allgemeinen eine auf den Mittelwert bezogene Standardabweichung von 90 - 95% angesehen. Je nach Mischaufgabe kann bereits eine kleinere Standardabweichung (< 90%) für den Rohrquerschnitt mit Verteiler ausreichend sein. Bei einer turbulenten Rohrströmung erfolgt eine zusätzliche Durchmischung stromabwärts des Verteilers durch turbulente Wirbelstrukturen und Diffusionsvorgänge in der Strömung. Die Mischaufgabe kann dann als zweistufiger Prozess betrachtet werden. Er besteht einerseits aus einer Vormischung des Fluids über den Rohrquerschnitt mit dem Verteiler und andererseits einer nachgeschalteten turbulenten Mischung im Rohr. Es ist bekannt, dass mit einer guten Vorverteilung der nachfolgende turbulente Mischungsvorgang in der Rohrströmung verbessert werden kann, so dass eine kürzere Rohrstrecke zum Erreichen einer guten Durchmischung notwendig ist. Für eine gute Vormischung sollte das aus dem Verteiler austretende Fluid einen möglichst großen Anteil des Rohrquerschnitts bei radialen Betrachtungsrichtungen direkt erfassen.

Um einen geringeren Druckverlust zu realisieren ist es erforderlich, die bekannten Mischvorrichtungen so zu konzipieren, dass ein größeres Volumen innerhalb des Verteilers mit dem beizumischenden Fluid 2 angefüllt ist, was sich jedoch ebenfalls nachteilig auf das Mischverfahren auswirkt.

Es stellte sich somit die Aufgabe, eine verbesserte Vorrichtung gemäß Anspruch 1 zur Vermischung zweier Fluidströme zu finden, welche die genannten Nachteile vermeidet und die eine verfahrenstechnisch einfache und effektive Vermischung bei hoher Verteilungswirkung ermöglicht. Weiterhin soll ein verbessertes Verfahren gemäß Anspruch 8 zur Vermischung von zwei Fluidströmen bereitgestellt werden.

Demgemäß wurde eine Vorrichtung zur Vermischung eines Fluidstromes 1 mit einem Fluidstrom 2 gefunden, welche ein Rohr umfasst, durch welches der Fluidstrom 1 geführt wird, wobei innerhalb des Rohres drei bis fünf Verteiler zur Zuleitung des Fluidstromes 2 angeordnet sind, welche in das Rohr hineinragen, wobei diese Anordnung der Verteiler durch Bohrungen im Mantel des Rohres ermöglicht wird und diese Verteiler einen freien Strömungsquerschnitt aufweisen, der in Hauptströmungsrichtung des Fluids 2 betrachtet sich stetig verkleinert und die Verteiler eine oder mehrere Lochreihen zur Zuleitung des Fluids 2 in das Fluid 1 aufweisen. Die Eintrittsstellen der Verteiler sind bevorzugt in einer Ebene senkrecht zur Längsachse des Rohres oder besonders bevorzugt in einem Abstand von maximal bis zum fünffachen des Rohrdurchmessers voneinander versetzt angeordnet.

Durch eine Anordnung von mindestens zwei Verteilern, welche radial in das Rohr, in welchem das Fluid 1 geführt wird, hineinragen, kann eine gute Verteilungswirkung bei relativ geringem volumenspezifischem Druckverlust erzielt werden. Zur näheren Erläuterung wird auf Figur 3 verwiesen, in welcher eine erfindungsgemäße Ausgestaltung der Vorrichtung exemplarisch dargestellt ist. Man sieht hier einen Schnitt durch ein das Fluid 1 führende Rohr (32), in welches drei Verteiler (31) radial hineinragen, wobei die Verteiler Bohrungen aufweisen, durch welche das den Verteilern aufgegebene Fluid 2 im Innenraum des Rohres (32) mit dem Fluid 1 vermischt wird. Im Vergleich zu der in Figur 1 dargestellten Vorrichtung ist es beim Betrieb der erfindungsgemäßen Vorrichtung vorteilhafterweise möglich, bei einem geringeren volumenspezifischen Druckverlust eine genauso gute Verteilungswirkung zu erzielen bzw. bei gleichem volumenspezifischen Druckverlust eine bessere Verteilungswirkung zu erreichen, wodurch sich der Betrieb der erfindungsgemäßen Vorrichtung als deutlich effektiver erweist. Dieser deutliche positive Effekt ist mit darauf zurückzuführen, dass bei der in Figur 3 dargestellten Vorrichtung die drei Verteiler separat von außen mit dem Fluid 2 beaufschlagt werden und durch diese Aufteilung des Stoffstromes anteilig betrachtet kürzere Lochreihenwege zu absolvieren sind wodurch es ermöglicht wird, mit einem geringeren Druckverlust die angestrebte Verteilungswirkung zu erzielen.

Die erfindungsgemäße Vorrichtung eignet sich für die Vermischung unterschiedlichster Fluidströme. So können beispielsweise mehrere gasförmige Fluide miteinander gemischt werden oder Fluide in flüssigem Aggregatzustand. Bevorzugt sind Mengenverhältnisse, bei denen ein oder mehrere kleinere Fluidströme 2 in den größeren Fluidstrom 1 über den Verteiler eingemischt wird. Bevorzugt ist ein Volumenverhältnis vom Fluidstrom 2 zum Fluidstrom 1 von 0,001 - 1. Besonders bevorzugt ist der Bereich von 0,01 - 0,5. Bevorzugte Stoffgruppen sind wässrige oder nichtwässrige anorganische oder organische Säuren und Laugen, sowie kurzkettige, aromatische , cyclische und cycloaliphatische Kohlenwasserstoffe sowie Metallkatalysatoren, metallorganische Katalysatoren oder organische Katalysatoren. Bevorzugt sind Fluide die miteinander unter Wärmefreisetzung reagieren. Besonders bevorzugt sind Cyclohexanderivate, die mit Oxidationsmitteln reagieren, so dass unter Wärmeentwicklung Oxidationsreaktionen ablaufen. Die Edukte dieser Reaktion können in einem der Fluidströme schon vorhanden sein. Besonders bevorzugt sind Wasser, Hydrogennitrat, Cyclohexanol ,Cyclohexanon sowie Kupfer- und Vanadiumkatalysatoren, die zusammen unter Wärmeentwicklung zu Adipinsäure sowie Glutarsäure und Bernsteinsäure reagieren. Dabei ist bevorzugt der Cyclohexanol/Cyclohexanon Strom der mengenmäßig kleinere Fluidstrom 2 und der wässrige Hydrogennitratstrom zusammen mit den Kupfer- und Vanadiumkatalysatoren der größere Fluidstrom 1 in den oben angegebenen Mengenverhältnissen.

Die erfindungsgemäß eingesetzten Verteiler weisen Öffnungen auf, durch welche das aufgegebene Fluid 2 austreten kann. Bevorzugt handelt es sich hierbei um kreisförmige Löcher, welche in Form von einer oder mehreren Reihen längs des Verteilers angeordnet sind. Die Durchmesser dieser Öffnungen bezogen auf den Eintrittsdurchmesser des Verteilers liegen bevorzugt in einem Bereich von 0,001 bis 0,5, besonders bevorzugt von 0,05 bis 0,2. Es empfehlen sich besonders drei bis 8 Lochreihen, wodurch eine weitere Vergleichmäßigung der Verteilung erzielt werden kann.

Unter dem freien Strömungsquerschnitt des Verteilers versteht man die Querschnittsfläche, welche dem Fluid 2 beim Durchströmen des Verteilers zur Verfügung steht. Diese Querschnittsfläche ist in Strömungsrichtung des Fluids 2 betrachtet veränderlich.

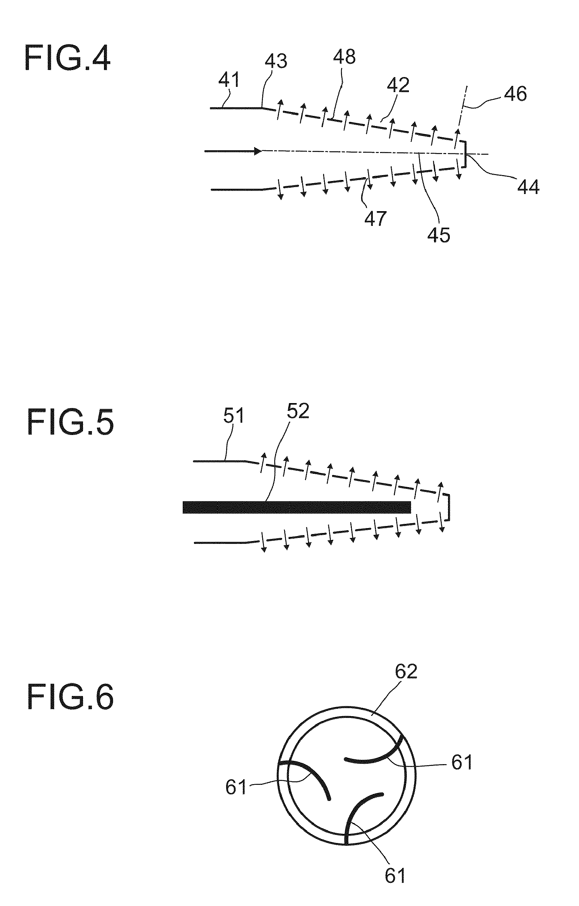

Eine bevorzugte Ausgestaltung des Verteilers ist in Figur 4 zu sehen. Der Verteiler (41) weist hier einen sich stetig verjüngenden Querschnitt auf (42). Weiterhin sind Öffnungen (47) (Löcher) zu sehen, durch welche das aufgegebene Fluid ausströmen kann. Eine Verringerung des freien Strömungsquerschnitts kann auch durch einen im Verteiler angebrachten Verdrängerkörper bewirkt werden. Dies ist in Figur 5 dargestellt. Man sieht hier einen Verteiler (51), der innen einen Verdrängerkörper (52) aufweist. Durch den verjüngenden Querschnitt wird vorteilhafterweise der sich ohne diese Maßnahme einstellende Anstieg der treibenden Druckdifferenz aufgrund des abnehmenden Massenstroms von Loch zu Loch durch die Erhöhung der Geschwindigkeit des im Verteiler verbleibenden Massenstroms gerade kompensiert. Die Erhöhung der Strömungsgeschwindigkeit im Verteiler entlang der Strömunsgrichtung kann durch einen konusförmigen Verteiler ohne Verdrängerkörper, einen zylinderförmigen Verteiler mit einem konusförmigen Verdrängerkörper, einen konusförmigen Verteiler mit einem zylindrischen Verdrängerkörper oder einen konusförmigen Verteiler mit einem konusförmigen Verdrängerkörper realisiert werden. Vorteilhaft ist bei einem konusförmigen Verteiler eine Abnahme der Querschnittsfläche in Strömungsrichtung. Die Querschnittsfläche im Verteiler kann bevorzugt ringförmig sein. Der Verdrängerkörper kann sich über die gesamte Länge oder nur einen Teil des Verteilers erstrecken. Bevorzugt werden bei den in das Rohr hineinragenden Verteilern in Hauptsrömungsrichtung des Fluids 2 betrachtet die freien Strömungsquerschnitte stetig verringert, wobei diese Verringerung durch unterschiedliche Maßnahmen bewirkt werden kann. Hierbei empfiehlt es sich, Verteiler mit einem kreisförmigen Querschnitt einzusetzen, welcher bevorzugt über die Hauptströmungsrichtung des Fluids 2 betrachtet gleich oder abnehmend ist. Hierbei kann der freie Strömungsquerschnitt innerhalb der Verteiler einen Ringspalt ausbilden.

Definitionen und bevorzugte Bereiche:

Die bevorzugte Ausführungsform des Verteilers hat eine gerade oder gebogene Geometrie, besonders bevorzugt dabei auch eine zylindrische oder kegelförmige Geometrie. Die folgenden Definitionen werden für die bevorzugte Ausführungsform vorgenommen und beschreiben Ausführungsformen mit elliptischen oder rechteckigen Querschnitten somit nicht eindeutig.

Unter dem volumenspezifischen Druckverlust im Sinne der vorliegenden Erfindung versteht man den Druckverlust für den durch den Verteiler strömenden Fluidstrom bezogen auf das freie Volumen eines zylinderförmigen Volumenelements an dem Verteiler mit folgenden Definitionen:

In Figur 3 wird der Durchmesser des Verteilers an der gemeinsamen Schnittfläche von Verteiler und Rohr (33) im Folgenden mit dem Symbol $D_0$ bezeichnet. Die zugehörige Fläche wird als Eintrittsquerschnitt bezeichnet. In dem Verteiler befindet sich das eingeschlossene Fluidvolumen $V_F$. Das Fluidvolumen im Verteiler ist definiert als das Volumen des Fluids (2), welches den im Verteiler zur Verfügung stehenden Rauminhalt bei verschlossenen Löchern bis zum Eintrittsquerschnitt einnimmt.

In Figur 3 ist das Rohr (32) dargestellt. Der zugehörige Durchmesser wird im Folgenden mit $D_R$ bezeichnet. Mit der Definition des Volumenverhältnisses wird das freie Volumen des Fluids in der Hauptströmung, welches nicht durch den Verteiler abgedeckt ist, und sich in einem zylindrischen Rohrelement mit der Länge $D_0$ und dem Durchmesser $D_R$ befindet, ins Verhältnis zum Gesamtvolumen des gleichen Rohrstücks ohne Verteiler gesetzt.

Das Volumenverhältnis $\varepsilon$ wird wie folgt definiert:

$$\varepsilon = \frac{\dfrac{\pi D_R{}^2}{4} D_0 - V_F}{\dfrac{\pi D_R{}^2}{4} D_0}$$

Zur Beschreibung vorteilhafter Geometrien und dem volumenspezifischen Druckverlust wird die dimensionslose Kennzahl DPV wie definiert:

$$DPV = \frac{\zeta}{\varepsilon} .$$

Druckverlust

$$\Delta p_v = \zeta \frac{\rho}{2} v^2$$

Dichte Fluid 2 ρ Druckverlustbeiwert ζ Geschwindigkeit im Eintrittsquerschnitt von Fluid 2 v

Ein Verteiler ist bei gleicher Aufteilung des Volumenstroms auf alle Löcher dann vorteilhaft, wenn der Wert von DPV klein ist. Bei einem Verteiler mit kleinem DPV Wert wird die für die gleichmäßige Aufteilung des Volumenstroms auf die einzelnen Löcher des Verteilers benötigte Energie bezogen auf das freie Volumen des Verteilers besser ausgenutzt, als bei einem Verteiler mit einem größeren DPV Wert.

Bei dem in Figur 4 dargestellten Verteiler ist die Länge zwischen dem Rand des Eintrittsquerschnitts (43) und dem Ende des Verteilers (44) dargestellt und wird im Folgenden mit $L_1$ bezeichnet.

$L_M$ ist die mit dem Rohrdurchmesser normierte Länge des Verteilers:

$$L_M = \frac{L_1}{D_R}$$

Die bevorzugte Länge des Verteilers bezogen auf den Rohrdurchmesser ist 0,05 bis 1, besonders bevorzugt 0,1 bis 0,5.

$D_{0,M}$ ist der mit dem Rohrdurchmesser normierte Durchmesser des Verteilers im Eintrittsquerschnitt.

$$D_{0,M} = \frac{D_0}{D_R}$$

Der bevorzugte normierte Durchmesser des Verteilers bezogen auf den Rohrdurchmesser ist von 0,005 bis 0,5, besonders bevorzugt von 0,01 bis 0,2.

Im Folgenden wird der Durchmesser des in Figur 4 dargestellten Verteilerendes (44) mit $D_1$ bezeichnet. Das dimensionslose Konus-Durchmesserverhältnis ist das Verhältnis von Enddurchmesser zum Eintrittsdurchmesser am Verteileranfang (43):

$$D_{1,M} = \frac{D_1}{D_0}$$

Das bevorzugte Konus-Durchmesserverhältnis des Verteilers ist 0 bis 1, besonders bevorzugt 0,25 bis 1.

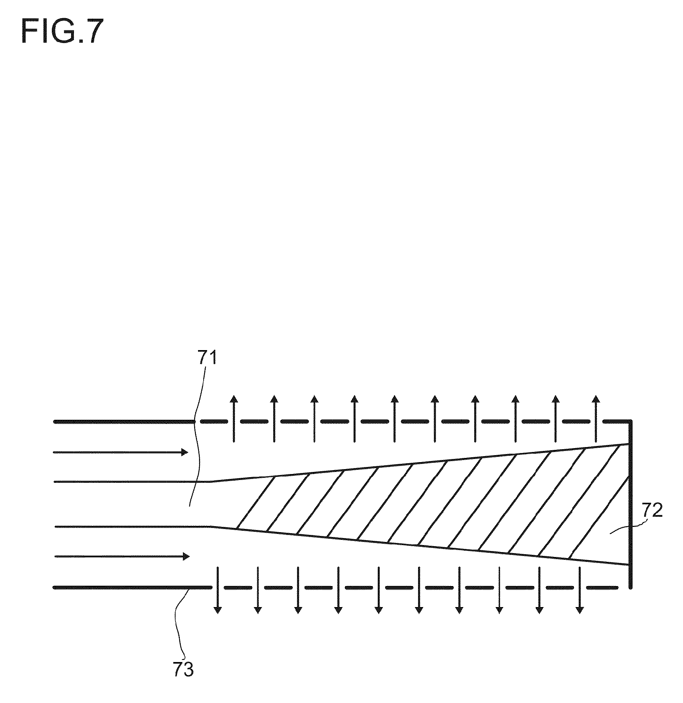

Bei dem in Figur 7 dargestellten Verteiler ist ein Verdrängerkörper (71) vorgesehen. Dieser hat am Eintrittsquerschnitt des Verteilers (73) den Durchmesser $D_{0,K}$. Der Durchmesser am Ende (72) des Verdrängerkörpers wird mit $D_{1,K}$ bezeichnet. Die Länge zwischen Anfang (73) und Ende (72) wird mit $L_{1,K}$ bezeichnet.. Die normierte Verdrängerkörper Länge ist:

$$L_K = \frac{L_{1,K}}{L_1}$$

Die bevorzugte Verdrängerkörperlänge ist von 0,1 bis 1, besonders bevorzugt von 0,3 bis 1.

$D_{0,K}$ ist der Aussendurchmesser des Verdrängerkörpers im Eintrittsquerschnitt. $D_{KM}$ ist das Durchmesserverhältnis von Verdrängerkörper und Verteiler:

$$D_{KM} = \frac{D_{0,K}}{D_0}$$

Das bevorzugte Durchmesserverhältnis von Verdrängerkörper und Verteiler ist von 0 bis 0,95, besonders bevorzugt von 0 bis 0,7.

$D_{1,K}$ ist der Durchmesser des Verdrängerkörpers an dem vom Eintrittsquerschnitt am weitesten entfernten Ende. Das normierte Durchmesserverhältnis des Verdrängerkörpers ist:

$$D_K = \frac{D_{0,K}}{D_{1,K}}$$

Das Verdrängerkörper-Durchmesserverhältnis ist bevorzugt von 0,1 bis 1, besonders bevorzugt von 0,3 bis 1.

Besonders bevorzugt ist eine konische Form des Verteilers. Dabei kann besonders bevorzugt im Strömungsquerschnitt stromaufwärts des Eintrittsquerschnitts in den Verteiler ein zylindrischer Verdrängerkörper vorhanden sein. Besonders bevorzugt ist ausserdem eine zylindrische Verteilergeometrie mit einem konischen Verdrängerkörper, bei dem wie in Figur 7 dargestellt der Enddurchmesser (72) größer ist als der Anfangsdurchmesser (71).

In einer bevorzugten Ausführungsform sind die Eintrittsstellen der Verteiler in das Rohr innerhalb einer Ebene senkrecht zur Längsachse des Rohres angeordnet. Diese Variante ist beispielsweise in Figur 3 zu sehen. Unter Eintrittstellen sind hierbei die Flächenschwerpunkte der Querschnittsflächen der Verteiler auf Höhe der Mantelfläche des Rohres (32) zu verstehen. Im Falle der hier dargestellten Verteiler mit kreisförmiger Querschnittsfläche befindet sich der Flächenschwerpunkt beispielsweise im Mittelpunkt dieser kreisförmigen Fläche und die Summe dieser Flächenschwerpunkte liegt somit auf der mittigen Längsache durch den Verteiler, wie sie durch eine Verlängerung der skizzierten Pfeile in den Verteiler hinein gebildet wird. Die Eintrittstelle liegt somit an der Schnittstelle dieser Achse mit der Mantelfläche (32) des Rohres und die hier in der Figur 3 dargestellten drei Eintrittstellen liegen in einer Ebene. Durch die Anordnung in einer Ebene befinden sich die Verteiler in unmittelbarer Nachbarschaft und hierdurch kann eine rasche und effektive Vermischung über den gesamten Querschnitt des Rohres (32) in kurzer Zeit gewährleistet werden. Dieser Effekt kommt besonders gut zum Tragen, wenn die Eintrittstellen der Verteiler bzw. die Verteiler in das Rohr tatsächlich innerhalb einer Ebene senkrecht zur Längsachse des Rohres angeordnet sind. Ein gewisser "Versatz" führt jedoch ebenfalls noch zu recht guten Ergebnissen und daher sind gemäß dieser Erfindung auch Eintrittstellen, welche in Bezug auf die Längsachse des Rohres (32) um bis zu dem 1,5 fachen des hydraulischen Durchmessers des Rohres (32) auf der Längsachse verschoben sind, von dieser Erfindung mit als "in einer Ebene liegend" umfasst. Unter hydraulischem Durchmesser versteht man hierbei den Quotienten aus dem vierfachen Strömungsquerschnitt und dem vom Fluid benetzten Umfang. Im Falle eines kreisförmigen Rohres (32) entspricht der hydraulische Durchmesser dem Rohrdurchmesser. Für andere Geometrien des Rohres (32) wie beispielsweise Kanäle mit rechteckigem Querschnitt kann der hydraulische Durchmesser leicht ermittelt werden.

Die erfindungsgemäßen Verteiler ragen wie beispielsweise in Figur 3 dargestellt in das Rohr (32) hinein. Dieses Prinzip bietet gegenüber bekannten Ringverteilern Vorteile, da sie beispielsweise einfach mittels Stutzen in das Rohr eingebracht werden können. Somit ist eine verfahrenstechnisch leichte Montage, Wartung und auch Nachrüstung möglich. Bevorzugt ragen 3 bis 5 Verteiler in das Rohr hinein, wobei bevorzugt mindestens ein Verteiler in seinem Inneren einen Verdrängungskörper aufweist.

Um eine effektive und gleichmäßige Verteilung zu gewährleisten, enthält die erfindungsgemässe Vorrichtung drei bis fünf Verteiler. Es ist auch vorgeschlagen, diese gleichmäßig über den Umfang des Rohres verteilt anzuordnen um die Gleichmäßigkeit der Durchmischung weiter zu erhöhen. Die für die Verteilung des Fluids 2 im Verteiler vorgesehenen Löcher können kreisförmig, elliptisch, rechteckig oder in Form eines Schlitzes ausgeführt werden.

Wie in Figur 4 dargestellt wird der Lochabstand (48) eingeführt als der Mittelpunktsabstand zweier benachbarter Öffnungen (47). Er wird im Folgenden mit dem Lochdurchmesser normiert und mit der Variable $s_L$ bezeichnet. Für runde Löcher liegt das Längenverhältnis $s_L$ aus Lochabstand und Lochdurchmesser bevorzugt zwischen 0,1 und 10, besonders bevorzugt zwischen 0,2 und 1,5.

In Figur 4 dargestellt ist die Mittelachse des Verteilers (45) sowie die Mittelachse eines Loches (46). Die Lochmittelachse (46) kann in Normalenrichtung zur Mittelachse des Verteilers (44) angeordnet sein oder um einen bestimmten Neigungswinkel, im Folgenden mit der Bezeichnung $\alpha_L$ benannt, davon abweichen. Der eingeschlossene Winkel zwischen der Mittelachse (45) und der Mittelachse (46) liegt bevorzugt zwischen 90 und 300°, besonders bevorzugt zwischen 90 und 70°.

Bezogen auf den Durchmesser $D_0$ des Verteilers liegt der Lochdurchmesser bevorzugt zwischen 0,01 und 0,5, besonders bevorzugt zwischen 0,05 und 0,2. Besonders bevorzugt sind Löcher mit Kreisförmigem Querschnitt oder mindestens ein flächengleicher, entlang der Längsachse angeordneter Schlitz, mit gleicher Querschnittsflächenverteilung in Abhängigkeit des Längenabstandes vom Eintrittsquerschnitt wie bei einer Anordnung mit Löchern. Der Lochdurchmesser kann bevorzugt entlang zumindest eines Verteilers in Hauptströmungsrichtung des Fluids (2) betrachtet innerhalb einer Lochreihe variieren. Bevorzugt ist eine Variation, bei der der Lochdurchmesser eines Loches bezogen auf den Lochdurchmesser des nächsten stromaufwärts liegenden, direkt benachbarten Lochs maximal um den Faktor 0,5-1,5 und besonders bevorzugt um den Faktor 0,8-1,2 variiert.

Wie vorstehend erläutert liegen die Eintrittstellen der Verteiler in der erfindungsgemäß definierten Art und Weise in einer Ebene. Die Verteiler können dann, wie in Figur 3 dargestellt, in Bezug auf ihre Längsachse linear in das Rohr (32) hineinragen.

Eine weitere bevorzugte Ausführungsform ist in Figur 6 dargestellt. Hier weisen die in das Rohr (62) hineinragenden Verteiler (61) eine gebogene Geometrie auf, wobei in einer besonders bevorzugten Ausführungsform die Krümmung der Verteiler derart ausgestaltet ist, dass sie hinsichtlich der vorstehend definierten charakteristischen Größe (Flächenschwerpunkt) vollumfänglich, also nicht nur in Bezug auf die Eintrittstelle in das Rohr (62), in einer Ebene senkrecht zur Längsachse des Rohres (62) angeordnet sind.

Bevorzugt weisen Verteiler wie auch das Rohr, in das sie hineinragen, einen kreisförmigen Querschnitt auf. Es können jedoch auch andere Geometrien wie beispielsweise rechteckige Querschnitte oder andere Formen Anwendung finden.

Die erfindungsgemäße Vorrichtung weist gegenüber dem Stand der Technik einen geringeren volumenspezifischen Druckverlust bzw. mit den vorgenommenen Definitionen geringeren DPV -Wert auf.

Die erfindungsgemäße Vorrichtung bietet eine effektive und verfahrenstechnisch einfache Art und Weise der Vermischung zweier oder mehrerer Fluide. Hier kann eine hohe Verteilungswirkung und gute Durchmischung bei relativ niedrigen volumenspezifischen Druckverlusten realisiert werden. Vorteilhafterweise kann die Vorrichtung leicht moniert und gewartet werden.

Beispiele

Es wird eine nicht erfindungsgemäße Vorrichtung nach dem bekannten Stand der Technik und eine erfindungsgemäße Vorrichtung miteinander verglichen. Beiden Vorrichtungen wird die gleiche Menge und das gleiche Fluid zugeführt. Dabei werden folgende Auslegungskriterien zu Grunde gelegt, um eine Vergleichbarkeit der Vorrichtungen bezüglich der Mischwirkung zu gewährleisten: die dimensionslose Länge $L_M$, die Anzahl der Löcher, der Lochabstand und der Durchmesser des Eintrittsquerschnitts $D_0$ sind für beide Vorrichtungen identisch. Als Fehlverteilung bei der Strömung im Verteiler und der Aufteilung des Gesamtvolumenstroms auf die einzelnen Löcher wird die Abweichung vom idealen Aufteilungszustand definiert, bei dem der Volumenstrom durch jedes Loch gleich groß ist. Für eine gute Vergleichbarkeit der hier betrachteten Mischorgane soll die Fehlverteilung auf die einzelnen Löcher ebenfalls identisch sein. Diese wird nach dem vorliegenden und allgemein bekannten Stand der Technik (Perry's Chemical Engineers Handbook, 8th Edition, Mc Graw Hill, 2008, p. 6-32 - 6-33) berechnet und mit 5% vorgegeben. Darauf basierend wird der Lochdurchmesser festgelegt. Die nicht erfindungsgemäße Vorrichtung, im Folgenden mit Variante (1) bezeichnet, besitzt eine zylindrische Form ohne weitere Einbauten. Die erfindungsgemäße Vorrichtung, im Folgenden mit Variante (2) bezeichnet, besitzt eine zylindrische Aussenform und einen konischen Verdrängerkörper. Beide Vorrichtungen haben jeweils vier über den Umfang verteilte Lochreihen und 31 Löcher pro Lochreihe. Für beide Varianten wird angenommen, dass jeweils drei Vorrichtungen radial in den Fluidstrom (1) eingeführt werden. In den tabellierten Ergebnissen ist zu erkennen, dass die Variante (2) bei gleicher Mischwirkung hinsichtlich des DPV Wertes vorteilhafter ist, da dieser kleiner ist.

| Geometriedefinitionen | Variante (1) | Variante (2) | |
|---|---|---|---|
| $L_M$ | 0,5 | 0,5 | [-] |
| $D_{0,M}$ | 0,05625 | 0,05625 | [-] |
| $D_{1,M}$ | - | 1 | [-] |
| $L_K$ | 1 | 1 | [-] |
| $D_{KM}$ | - | 0,556 | [-] |
| $D_K$ | - | 0,667 | [-] |
| Normierter Lochdurchmesser | 0,0588 | 0,0806 | [-] |
| Winkel $\alpha_L$ | 90° | 90° | ° |
| Abstand $s_L$ | 1,97 | 1,78 | [-] |
| $\varepsilon$ | 0,9873 | 0,9935 | [-] |
| $\zeta$ | 10,1 | 3,1 | [-] |
| **DPV** | **10,2** | **3,1** | [-] |

Definition und Gültigkeitsbereich des Beispiels:

Es wird mit der Viskosität $\eta$ des Fluids, der Dichte $\rho$ des Fluids, dem Eintrittsdurchmesser $D_0$ des Verteilers und der zugehörigen Eintrittsgeschwindigkeit $u_0$ im Eintrittsquerschnitt die Reynolds-Zahl Re wir folgt definiert:

$$\mathrm{Re} = \frac{\rho\, u_0\, D_0}{\eta}.$$

Obiges Beispiel gilt für einen Einsatzbereich des Verteilers mit einer Reynolds-Zahl Re größer als 2300.

**Patentansprüche**

1. Vorrichtung zur Vermischung eines Fluistromes 1 mit einem Fluidstrom 2 umfassend ein Rohr, durch welches der Fluidstrom 1 geführt wird, wobei innerhalb des Rohres Verteiler zur Zuleitung des Fluidstromes 2 angeordnet sind, wobei diese Anordnung der Verteiler durch Bohrungen im Mantel des Rohres ermöglicht wird und diese Verteiler einen freien Strömungsquerschnitt aufweisen, der in Hauptströmungsrichtung des Fluids 2 betrachtet sich stetig verkleinert und die Verteiler eine oder mehrere Lochreihen zur Zuleitung des Fluids 2 in das Fluid aufweisen, **dadurch gekennzeichnet, dass** drei bis 5 Verteiler angeordnet sind, welche in das Rohr hineinragen, und dass die Verteiler gleichmäßig über den Umfang des Rohres verteilt angeordnet sind.

2. Vorrichtung nach Anspruch 1, wobei die Eintrittstellen der Verteiler in das Rohr innerhalb einer Ebene senkrecht zur Längsachse des Rohres angeordnet sind.

3. Vorrichtung nach Anspruch 2, wobei die Verteiler innerhalb einer Ebene senkrecht zur Längsachse des Rohres angeordnet sind.

4. Vorrichtung nach Anspruch 1 bis 3, wobei die in das Rohr hineinragenden Verteiler in Hauptströmungsrichtung des Fluids 2 betrachtet eine stetige Verringerung des freien Strömungsquerschnitts aufweisen.

5. Vorrichtung nach Ansprüchen 1 bis 4 umfassend mindestens einen Verteiler, bei welchem die Durchmesser der Löcher innerhalb einer Lochreihe in Hauptströmungsrichtung des Fluids 2 betrachtet variiert werden.

6. Vorrichtung nach Ansprüchen 1 bis 5 wobei die Verteiler einen kreisförmigen Querschnitt aufweisen, der über die Hauptströmungsrichtung des Fluids 2 betrachtet gleich oder abnehmend ist.

7. Vorrichtung nach Ansprüchen 1 bis 6 wobei das Rohr einen kreisförmigen Querschnitt aufweist.

8. Verfahren zur Vermischung zweier Fluidstrome 1 und 2, **dadurch gekennzeichnet, dass** man eine Vorrichtung gemäß Ansprüchen 1 bis 7 einsetzt.

**Claims**

1. An apparatus for the intermixing of a fluid stream 1 with a fluid stream 2, comprising a pipe through which the fluid stream 1 is routed, distributors for delivering the fluid stream 2 being arranged inside the pipe, this arrangment of the distributors being made possible by bores in the casing of the pipe, and these distributors having a free flow cross section which, as seen in the main direction of flow of the fluid 2, decreases continuously and the distributors having one or more rows of holes for delivering the fluid 2 into the fluid, wherein three to five distributors are provided which project into the pipe and wherein the distributors are arranged uniformly over the circumference of the pipe.

2. The apparatus according to claim 1, the inlet points of the distributors into the pipe being arranged within a plane perpendicular to the longitudinal axis of the pipe.

3. The apparatus according to claim 2, the distributors being arranged within a plane perpendicular to the longitudinal axis of the pipe.

4. The apparatus according to claims 1 to 3, the distributors which project into the pipe having a continuous reduction in the free flow cross section, as seen in the main direction of flow of the fluid 2.

5. The apparatus according to claims 1 to 4, comprising at least one distributor in which the diameters of the holes are varied within a row of holes, as seen in the main direction of flow of the fluid 2.

6. The apparatus according to claims 1 to 5, the distributors having a circular cross section which is identical or decreasing, as seen in the main direction of flow of the fluid 2.

7. The apparatus according to claims 1 to 6, the pipe having a circular cross section.

8. A method for the intermixing of two fluid streams 1 and 2, wherein an apparatus according to claims 1 to 7 is used.

**Revendications**

1. Dispositif de mélange d'un courant de fluide 1 avec un courant de fluide 2, comportant un tube à travers lequel le courant de fluide 1 est guidé, des distributeurs étant disposés à l'intérieur du tube pour l'amenée du courant de fluide 2, cet agencement des distributeurs étant rendu possible par des alésages dans l'enveloppe du tube et ces distributeurs présentant une section transversale d'écoulement libre qui, vue dans la direction d'écoulement principale du fluide 2, se rétrécit de manière continue et les distributeurs comprenant une ou plusieurs rangées de trous pour l'amenée du fluide 2 dans le fluide, **caractérisé en ce que** de trois à 5 distributeurs sont prévus, lesquels pénètrent dans le tube, et **en ce que** les distributeurs sont agencés de manière distribuée régulièrement sur la périphérie du tube.

2. Dispositif selon la revendication 1, dans lequel les points d'entrée des distributeurs dans le tube sont agencés à l'intérieur d'un plan perpendiculaire à l'axe longitudinal du tube.

3. Dispositif selon la revendication 2, dans lequel les distributeurs sont agencés à l'intérieur d'un plan perpendiculaire à l'axe longitudinal du tube.

4. Dispositif selon les revendications 1 à 3, dans lequel les distributeurs pénétrant dans le tube présentent, vus dans la direction d'écoulement principale du fluide 2, un rétrécissement continu de la section transversale d'écoulement libre.

5. Dispositif selon les revendications 1 à 4, comportant au moins un distributeur, dans lequel le diamètre des trous à l'intérieur d'une rangée de trous varie vu dans la direction d'écoulement principale du fluide 2.

6. Dispositif selon les revendications 1 à 5, dans lequel les distributeurs présentent une section transversale circulaire qui, vue dans la direction d'écoulement principale du fluide 2, est constante ou décroissante.

7. Dispositif selon les revendications 1 à 6, dans lequel le tube présente une section transversale circulaire.

8. Procédé de mélange de deux courants de fluide 1 et 2, **caractérisé en ce qu'**on utilise un dispositif selon les revendications 1 à 7.

FIG.1

FIG.2

FIG.3

FIG.4

FIG.5

FIG.6

# FIG.7

**IN DER BESCHREIBUNG AUFGEFÜHRTE DOKUMENTE**

**In der Beschreibung aufgeführte Patentdokumente**

- DE 102008037374 A1
- DE 102007054770 A1
- EP 1354866 B1
- FR 1211889 A

**In der Beschreibung aufgeführte Nicht-Patentliteratur**

- **PAUL, E.L.** Handbook of Industrial Mixing. Jon Wiley & Sons, 2004, 391-452
- Perry's Chemical Engineers Handbook. Mc Graw Hill, 2008, 6-32, 6-33
- **CHEN, A.W. ; SPARROW, E.M.** *Journal Fluid. Eng.,* 2009, vol. 131, 1-9